(19) Europäisches Patentamt
European Patent Office
Office européen des brevets

(11) **EP 3 185 011 B1**

(12) **FASCICULE DE BREVET EUROPEEN**

(45) Date de publication et mention de la délivrance du brevet:
**11.09.2019 Bulletin 2019/37**

(51) Int Cl.:
**G01N 33/00** *(2006.01)* **G01N 25/48** *(2006.01)*
**G01N 5/04** *(2006.01)*

(21) Numéro de dépôt: **16206507.2**

(22) Date de dépôt: **22.12.2016**

(54) **CAPTEUR DE GAZ MICROELECTROMECANIQUE OU NANOELECTROMECANIQUE**

MIKRO- ODER NANOELEKROMECHANISCHER GASSENSOR

MICROELECTROMECHANICAL OR NANOELECTROMECHANICAL GAS SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2015 FR 1563217**

(43) Date de publication de la demande:
**28.06.2017 Bulletin 2017/26**

(73) Titulaire: **COMMISSARIAT À L'ÉNERGIE ATOMIQUE ET AUX ÉNERGIES ALTERNATIVES**
**75015 Paris (FR)**

(72) Inventeurs:
• **TOFFOLI, Valeria**
**80803 München (DE)**
• **ALAVA, Thomas**
**38000 GRENOBLE (FR)**
• **BOURLON, Bertrand**
**38950 SAINT MARTIN LE VINOUX (FR)**
• **OLLIER, Eric**
**38100 GRENOBLE (FR)**

(74) Mandataire: **Brevalex**
**95, rue d'Amsterdam**
**75378 Paris Cedex 8 (FR)**

(56) Documents cités:
**US-A1- 2008 085 212      US-A1- 2010 055 801**

• **Matthew Kasper: "SIMULTANEOUS NANOTHERMAL ANALYSIS USING HEATED MICROCANTILEVERS", , 30 avril 2010 (2010-04-30), XP055371249, Urbana, USA Extrait de l'Internet: URL:https://www.ideals.illinois.edu/bitstr eam/handle/2142/16213/Kasper_Matthew.pdf?s equence=3&isAllowed=y [extrait le 2017-05-10]**
• **VALERIA TOFFOLI ET AL: "Heater-Integrated Cantilevers for Nano-Samples Thermogravimetric Analysis", SENSORS, vol. 13, no. 12, 4 décembre 2013 (2013-12-04), pages 16657-16671, XP055371168, DOI: 10.3390/s131216657**
• **RÜDIGER BERGER ET AL: "Thermal Analysis of Nanogram Quantities Using a Micromechanical Cantilever Sensor", NATAS 2002 CONFERENCE PITTSBURGH (USA), 1 août 2002 (2002-08-01), pages 23-25, XP055371183,**
• **ELINA IERVOLINO ET AL: "MEMS for Thermogravimetry: Fully Integrated Device for Inspection of Nanomasses", JOURNAL OF MICROELECTROMECHANICAL SYSTEMS, IEEE SERVICE CENTER, US, vol. 20, no. 6, 1 décembre 2011 (2011-12-01), pages 1277-1286, XP011379423, ISSN: 1057-7157, DOI: 10.1109/JMEMS.2011.2167672**

**Description**

**DOMAINE TECHNIQUE ET ÉTAT DE LA TECHNIQUE ANTÉRIEURE**

La présente invention se rapporte à un capteur de gaz offrant une sélectivité améliorée et une compacité augmentée et à un procédé d'analyse mettant en oeuvre ledit capteur de gaz.

On cherche de plus en plus à vérifier la composition gazeuse de l'environnement pour, par exemple, détecter des problèmes de pollution. En moyenne, les êtres humains passent plus de 80 % de leur vie à l'intérieur, on cherche alors à contrôler la qualité de l'air intérieur, notamment du fait de l'utilisation de nouveaux matériaux chimiques et synthétiques dans la construction.

De nombreux capteurs sont capables de réaliser des détections quantitatives d'espèces gazeuses sous forme de trace. Il existe des capteurs qui détectent une modification d'une propriété physique de l'environnement extérieur, comme c'est le cas des détecteurs de conductivité thermique, qui sont sensibles à une modification du coefficient de conductivité thermique de l'environnement extérieur. Il existe également des capteurs qui sont sensibles à une adsorption d'espèces gazeuses sur une surface, ce type de détecteur comporte une surface sensible qui favorise l'adsorption et /ou la désorption.

Cependant, il n'existe pas de capteurs qui ne soient sensibles qu'à une seule espèce gazeuse. Il en résulte que pour discriminer les espèces gazeuses contenues dans le mélange, le capteur est associé à un dispositif capable de séparer dans le temps et/ou dans l'espace les espèces gazeuses afin de décoreller les contributions des différentes espèces dans la réponse du capteur.

Par exemple, on utilise des filtres ou des colonnes de chromatographie associées ou non à des pré-concentrateurs pour assurer cette séparation. Une colonne de chromatographie assure la séparation de chacune des espèces mais présente l'inconvénient d'être difficilement intégrable pour former un dispositif d'analyse compact. En outre elle requiert une certaine quantité d'énergie thermique pour son fonctionnement ainsi qu'une alimentation en gaz porteur. Il existe des microcolonnes en phase gazeuse qui peuvent être intégrées sur une puce en silicium mais elles exigent toujours un chauffage important et une source de gaz porteur.

Concernant les dispositifs de pré-concentration, ceux-ci permettent la détection de traces mais ils nécessitent en général un volume important, notamment pour maximiser le nombre d'espèces collectées. Ils requièrent également un chauffage qui doit présenter un bonne dynamique pour assurer une libération rapide des espèces pré-concentrées, en particulier lorsqu'ils sont couplés à une colonne de chromatographie.

Ces dispositifs de séparation sont donc encombrants et consommateurs d'énergie.

Le document WO01/40793 décrit un dispositif comportant un matériau apte à absorber et désorber des gaz, un moyen de chauffage de ce matériau et un capteur de gaz sélectif. Les espèces adsorbées par le matériau sont désorbées par chauffage et sont ensuite détectées par le capteur de gaz sélectif. Ce dispositif est encombrant.

Le document US 2005/0090018 décrit un dispositif de détection des composés organiques volatiles ou VOCs pour Volatile Organic compounds en terminologue anglo-saxonne en forte concentration, comportant une membrane adsorbante et un capteur de gaz situés l'un à côté d l'autre. Un moyen de chauffage applique un choc thermique à la membrane, les espèces désorbées traversent la membrane et sont détectées par le capteur. Ce dispositif est également encombrant.

Le document Matthew Kasper: "SIMULTANEOUS NANOTHERMAL ANALYSIS USING HEATED MICROCANTILEVERS", 30 avril 2010 (2010-04-30), XP055371249,Urbana, USA décrit une structure MEMS comprenant une partie fixe, une partie suspendue avec une zone sensible, des moyens de chauffage et des moyens de détection de l'adsorption/absorption et de la désorption en fonction de la température.

Par ailleurs, on cherche à pouvoir intégrer les capteurs de gaz dans des dispositifs électroniques portables tels que des téléphones portables ou des assistants numériques personnels pour par exemple détecter un environnement sûr ou alors présentant des polluants.

**EXPOSÉ DE L'INVENTION**

C'est par conséquent un but de la présente invention d'offrir un capteur de gaz compact tout en offrant une sélectivité améliorée.

Le but énoncé ci-dessus est atteint par un capteur comportant une partie suspendue par rapport à une partie fixe, au moins une zone sensible sur la partie suspendue, ladite zone sensible étant apte à adsorber/absorber et à désorber au moins deux espèces gazeuses, des moyens de détection de l'adsorption et de la désorption de les au moins deux espèces gazeuses, des moyens de chauffage de ladite zone sensible et des moyens de contrôle des moyens de chauffage de sorte à appliquer un ou des profils de température à la zone sensible afin de générer au moins une phase d'adsorption et une phase de désorption.

On entend par « profil de température » une évolution de la température appliquée à la zone sensible en fonction du temps. Ce profil peut par exemple comporter une phase de maintien à température constante pendant une durée donnée provoquant l'adsorption et une autre phase de maintien à température constante à une température supérieure provoquant la désorption, ou des phases de variations de la température par palier ou en créneau ou en continue de manière monotone ou non, il peut s'agir d'une variation sinusoïdale à fréquence constante ou variable ou d'impulsions.

En choisissant le ou les profils de température, il est possible d'améliorer la sélectivité du capteur vis-à-vis de certaines espèces gazeuses, par exemple en adsorbant/absorbant toutes les espèces gazeuses puis en désorbant une à une les espèces gazeuses, l'adsorption puis les désorptions successives peuvent être détectées par exemple par mesure d'une variation de résistance électrique de la zone sensible ou par détection d'une variation de fréquence de résonance de la partie suspendue.

En d'autres termes, on utilise la ou les zones sensibles du capteur dont la température est contrôlée comme dispositif de séparation des espèces gazeuses. On réalise un contrôle en temps réel des interactions stationnaires et / ou dynamique entre les espèces contenues dans le gaz et la ou les zones sensibles pour assurer leur séparation.

Le capteur selon l'invention présente alors une grande sélectivité et une grande compacité car il intègre les moyens de séparation, contrairement à certains capteurs qui requiert d'être associés à une colonne de chromatographie en phase gazeuse qui préalablement sépare les espèces gazeuses qui sont ensuite détectées par le capteur.

Le capteur selon l'invention intègre donc à la fois la fonction de collecte, de séparation et de détection des espèces gazeuses. Il présente donc une capacité d'identification augmentée.

De manière avantageuse, le capteur selon l'invention peut être intégré à un système de détection et être associé à d'autres capteurs de types MEMS et/ou NEMS pour améliorer la sélectivité du système de détection, par exemple en corrélant les signaux émis par les différents capteurs.

Dans un exemple très avantageux, les capteurs du système de détection peuvent interagir entre eux en en s'échangeant les analytes gazeux. Ils peuvent collecter, détecter et séparer les espèces du mélange gazeux de façon complémentaire et spécifique. Certains des analytes peuvent être détectés par plusieurs capteurs séquentiellement. En variante, les capteurs peuvent être utilisés de la même façon formant par exemple un réseau de plusieurs capteurs ciblant des espèces gazeuses différentes.

La présente invention a alors pour objet un capteur de gaz microélectromécanique et/ou nanoélectromécanique selon la revendication 1.

Avantageusement le capteur de gaz comporte des moyens de mesure de la température de la zone sensible.

Dans un exemple de réalisation, la partie suspendue est mobile et les moyens de détection comportent des moyens d'actionnement pour mettre en mouvement la partie suspendue et des moyens pour mesurer le déplacement de la partie suspendue.

Dans un autre exemple de réalisation, la partie suspendue est au moins en partie conductrice électrique et les moyens de détection mesurent une variation de conductivité de la partie suspendue.

Les moyens de chauffage peuvent être des moyens de chauffage à effet joule. La partie suspendue peut alors être au moins en partie conductrice électrique et être destinée à s'échauffer par effet Joule par circulation d'un courant.

Très avantageusement, les moyens de détection et les moyens de chauffage sont confondus.

Le capteur peut comporter une base de données comportant des profils de température prédéfinis.

Selon une caractéristique additionnelle, le capteur peut comporter plusieurs parties suspendues comportant des zones sensibles de surface différentes et/ou ayant des propriétés d'adsorption/absorption et désorption différentes les unes des autres.

Dans un exemple, les moyens de chauffage sont tels qu'ils peuvent chauffer différentes zones de la partie mobile à des températures différentes simultanément.

La présente invention a également pour objet un système microélectromécanique et/ou nanoélectromécanique de détection de gaz comportant au moins un premier capteur de gaz selon l'invention et au moins un deuxième capteur de gaz et des moyens de comparaison et de traitement des signaux issus des moyens de détection de chacun desdits capteurs.

Le deuxième capteur de gaz peut être un capteur de gaz selon l'invention. Dans un exemple de réalisation, le premier capteur de gaz est disposé dans un environnement de référence et le deuxième capteur étant destiné à être en contact avec un mélange gazeux.

Dans un autre exemple, le premier capteur de gaz et le deuxième capteur de gaz peuvent être disposés dans un même mélange gazeux, au moins une zone sensible du premier capteur de gaz étant apte à adsorber/absorber et à désorber une première espèce gazeuse ou première famille d'espèce gazeuse à détecter et le deuxième capteur de gaz étant un capteur de gaz selon l'invention, au moins une zone sensible du deuxième capteur étant apte à adsorber/absorber et à désorber une deuxième espèce gazeuse ou deuxième famille d'espèce gazeuse à détecter.

La première espèce gazeuse ou première famille d'espèce gazeuse et la deuxième espèce gazeuse ou deuxième famille d'espèce gazeuse peuvent comporter des éléments gazeux communs ou n'avoir aucun élément gazeux en commun.

Le capteur peut comporter plusieurs deuxièmes capteurs disposés autour du premier capteur, les moyens de contrôle des deuxièmes capteurs étant tels qu'ils adsorbent/absorbent les espèces gazeuses ou familles d'espèces désorbées par le premier capteur.

Dans un exemple, le système comporte un premier capteur et un deuxième capteur disposé en amont du premier capteur et un autre deuxième capteur disposé en aval du premier capteur et des moyens de comparaison des signaux fournis par les moyens de détection de deux deuxièmes capteurs.

Les capteurs peuvent comporter des zones sensibles différentes.

Le système peut comporter plusieurs premiers capteurs et les deuxièmes capteurs peuvent être répartis en plusieurs groupes agencés de manière concentrique autour des premiers capteurs, les moyens de contrôle des deuxièmes capteurs étant tels qu'ils contrôlent les moyens de chauffage de sorte que les zones sensibles de chaque groupe soient à une température différente de celles des autres groupes.

De manière avantageuse, le système comporte un canal dans lequel est ou sont montés les capteurs

Le système peut comporter une pompe connectée au canal.

La présente invention a également pour objet un procédé d'analyse d'un mélange gazeux mettant en oeuvre un capteur selon l'invention ou un système de détection selon l'invention, comportant au moins un cycle comprenant les étapes :

a) application à au moins une la zone sensible d'une première température pour provoquer une adsorption/absorption d'espèces gazeuses,
b) mesure d'un signal de détection de l'adsorption/absorption,
c) application d'une deuxième température pour provoquer la désorption d'au moins une partie des espèces gazeuses,
d) mesure d'un signal de détection de ladite désorption,
e) répétition des étapes a), b), c) et d) avec des températures d'adsorption/absorption différentes de celles de l'étape a) précédente jusqu'à ce que toutes les espèces d'intérêt soient séparées,

Dans un mode de fonctionnement avantageux, les conditions d'adsorption/absorption et de désorption du cycle suivant sont déterminées en fonction des signaux obtenus à l'étape d).

Dans un mode de fonctionnement avantageux, les conditions de désorption de l'étape c) sont déterminées en fonction des signaux obtenus à l'étape b).

De préférence, le procédé comporte au moins préalablement à l'étape a), au moins une étape de nettoyage par application d'une température provoquant une désorption telle que la zone sensible se retrouve dans l'état de référence. De manière préférée, le procédé comporte une étape de nettoyage entre chaque cycle.

Dans un mode de fonctionnement, lorsque la valeur du signal de détection à l'étape b) est inférieure à une valeur seuil, le procédé d'analyse est interrompu.

## BRÈVE DESCRIPTION DES DESSINS

La présente invention sera mieux comprise sur la base de la description qui va suivre et des dessins en annexe sur lesquels :

- la figure 1 est une représentation schématique d'un exemple de capteur selon l'invention,
- les figures 2A à 2F sont des représentations graphiques d'exemples de profils de température pouvant être appliqués à la zone sensible du capteur,
- la figure 3 est une représentation en perspective d'un exemple d'une zone sensible pouvant être mise en oeuvre dans un capteur selon l'invention,
- la figure 4 est une représentation en perspective d'un autre exemple de réalisation d'une zone sensible pouvant être mise en oeuvre dans un capteur selon l'invention,
- la figure 5 est une représentation schématique d'une vue de dessus d'un système de détection mettant en oeuvre plusieurs capteurs selon un exemple de réalisation,
- la figure 6 est une représentation schématique d'une vue de dessus d'un système de détection mettant en oeuvre plusieurs capteurs selon un autre exemple de réalisation,
- la figure 7 est une vue en coupe longitudinale d'un exemple de réalisation d'un système de mesure à plusieurs capteurs selon l'invention montés dans un canal fluidique,
- la figure 8 est une vue en coupe transversale d'un autre exemple de réalisation d'un système de détection mettant en oeuvre plusieurs capteurs selon l'invention dans un canal fluidique,

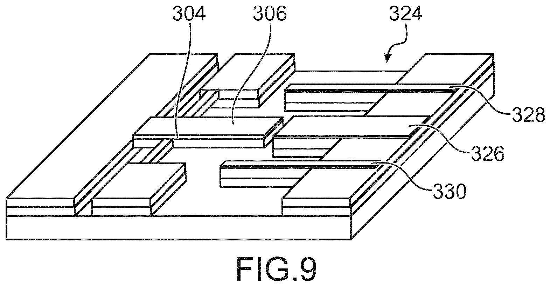

- la figure 9 est une vue en perspective de système de détection comportant un capteur selon l'invention et un capteur d'un autre type,
- la figure 10 est une représentation graphique d'un exemple de profil de température appliqué à un capteur selon l'invention et du signal émis par les moyens de détection en fonction du temps,

- les figures 11A à 11H sont des représentations schématiques de différentes étapes d'un exemple d'un procédé de fabrication d'un capteur selon l'invention.

## EXPOSÉ DÉTAILLÉ DE MODES DE RÉALISATION PARTICULIERS

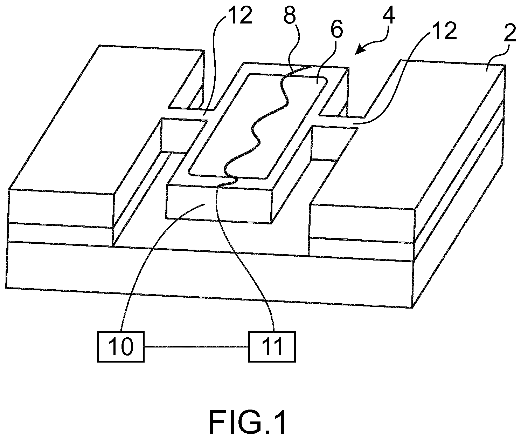

Sur la figure 1, on peut voir une représentation schématique d'un exemple de capteur de gaz selon l'invention.

Le capteur de gaz est destiné à détecter les espèces gazeuses contenues dans un mélange gazeux. Ces espèces sont potentiellement présentes dans le mélange gazeux, on cherche alors à détecter l'apparition de chacune d'elles et leur concentration. Par exemple, on cherche à détecter l'apparition d'une espèce dangereuse, par exemple polluante cette espèce étant identifiée. Le capteur est donc adapté à la détection de certaines espèces, notamment au niveau de la zone sensible et des moyens de contrôle de la température de la zone sensible. De préférence le capteur est réalisé à partir d'un dispositif microélectromécanique (MEMS pour Microlectromechanical Systems en terminologie anglo-saxonne) et/ou un dispositif nanoélectromécanique (NEMS pour Microlectromechanical Systems en terminologie anglo-saxonne).

Le capteur comporte un partie fixe 2, une partie 4 suspendue par rapport à la partie fixe 2, une zone sensible 6 portée par la partie suspendue 4, des moyens de chauffage 8 d'au moins la zone sensible 6.

La zone sensible 6 est apte à adsorber/absorber et désorber des espèces gazeuses, également appelées analytes. Dans la suite de la description, le terme « adsorption » sera utilisé pour désigner l'adsorption et l'absorption.

Le capteur comporte également des moyens 10 pour détecter les changements physico-chimiques au sein de la zone sensible provoqués par l'adsorption et la désorption des espèces gazeuses sur la zone sensible. Les moyens de détection 10 seront décrits par la suite.

La mise en oeuvre d'une partie suspendue permet de limiter les pertes thermiques et de localiser le chauffage, l'énergie requise pour le chauffage de la zone sensible est alors avantageusement réduite.

La partie suspendue est par exemple sous forme de membrane ou sous forme de pont suspendu par rapport à la partie fixe, par exemple au moyen de poutres 12 de section faible afin de limiter encore davantage les fuites thermiques de la partie suspendue 4 à la partie fixe et de localiser le chauffage. La membrane peut être supportée par un ou plusieurs piliers.

La partie suspendue peut être en forme de disque suspendu par des poutres. En variante, la partie suspendue est de type nanofil ou poutre encastrée, de préférence la forme et la section de la poutre sont optimisées pour améliorer la sensibilité de détection. A titre d'exemple avantageux, dans le cas d'une partie suspendue formée par une poutre suspendue, la poutre pourrait avoir une section de 160 nm par 200 nm.

La zone sensible 6 comporte une couche formée sur au moins l'une des faces de la partie suspendue, la couche recouvre tout ou partie de l'une ou des deux faces de la partie suspendue. De préférence, la couche a une épaisseur sensiblement constante. Le choix du matériau de la couche formant la zone sensible est choisi en fonction des espèces que l'on cherche à détecter dans le mélange à analyser.

La couche sensible comporte au moins un matériau apte à adsorber les espèces d'intérêt. La couche sensible peut ne comporter qu'un seul matériau ou être enrichie. Le choix de la couche sensible dépend des espèces d'intérêt à analyser, par exemple s'il s'agit d'espèce organique ou inorganique...

Par exemple, il peut s'agir d'une résine polymère poreuse, telle que le Tenax TA®, Tenax GR®, Carbotrap®, Carboxen®, Carbosieve®, qui est utilisée pour détecter des composés organiques volatiles ou semi-volatiles et discriminer les composés.

Selon d'autres exemples, il peut s'agir :

- de poudre de silice mésoporeuse avec des canaux orientés en deux directions telle que la SBA - Santa Barbara Amorphous -and la MCM - Mobil Crystalline Materials en terminologie anglo-saxonne, ou en silicium poreux,
- d'alumine ($Al_2O_3$) avec des pores de différentes dimensions ou avec des pores fonctionnalisés par des radicaux chimiques offrant des propriétés physico-chimiques differentes, particulièrement adapté pour l'adsorption des VOCs,
- du matériau poreux formant la partie suspendue du MEMS et/ou NEMS, par exemple du silicium poreux,
- de nanotubes de carbone, à la fois les nanotubes de carbone monoparois (Single-walled carbon nanotubes (SWNTs) en terminologie anglo-saxonne) et les nanotubes de carbone multi-parois (multi-walled carbon nanotubes (MWNTs) en terminologie anglo-saxonne) qui sont efficaces pour adsorber une large variété de polluants (merci de donner un exemple de polluant).

Le capteur comporte également des moyens de contrôle des moyens de chauffage de sorte à pouvoir appliquer des profils de température à la zone sensible en fonction du mélange gazeux à analyser.

Les moyens de chauffage peuvent être du type à effet Joule, le courant électrique circulant dans la partie suspendue qui est en matériau tel que le passage du courant provoque un échauffement, par exemple un fil conducteur électrique serpentant sur la partie suspendue provoquerait l'échauffement de la zone sensible. En variante, il peut s'agir de moyens de chauffage par rayonnement thermique, ceux-ci étant alors par exemple disposés sur la partie fixe en regard de la partie suspendue. D'autres techniques de chauffage peuvent être utilisées, par exemple ils peuvent mettre en oeuvre des structures électriques utilisant les effets Peltier et Seebeck.

Avantageusement, une couche de diffusion thermique peut être prévue sur la partie suspendue pour assurer une température homogène de la zone sensible.

De manière très avantageuse, le capteur comporte des moyens de mesure de la température au niveau de la zone sensible afin de pouvoir commander au mieux les moyens de chauffage et maîtriser les différentes étapes d'analyse. Par exemple ces moyens de mesure comportent un thermocouple disposé sur la partie suspendue de sorte à mesurer la température de la zone sensible. En mesurant la résistance du fil conducteur formant le thermocouple on connaît la température de la zone sensible. En variante les moyens de mesure peuvent être du type sans contact, par exemple il peut s'agir de moyens de lecture infrarouge ou laser.

Les moyens de détection peuvent être de plusieurs types.

Dans un mode de réalisation, la partie suspendue est mobile.

Les moyens de détection peuvent être de type micro gravimétrique résonant. Pour cela la partie suspendue est mise en mouvement sous l'action de moyens d'excitation à sa fréquence de résonance. Lorsqu'une ou des espèces gazeuses s'adsorbe(nt) ou désorbe(nt) sur la zone sensible ou désorbe(nt), la fréquence de résonance varie. En mesurant cette variation, on peut détecter le ou les espèces adsorbées ou désorbées et leur quantité.

Les moyens d'excitation ou de déplacement de la partie suspendue sont par exemple du type électrostatique, magnétique, piézoélectrique, opto-mécanique ou thermique.

Les moyens de mesure du déplacement sont par exemple de type électrique, électrostatique, piézorésistif, piézoélectrique ou magnétique.

Dans un autre mode de réalisation dans lequel la partie suspendue est fixe, les moyens de détection peuvent être du type résistif, i.e. la zone sensible est par exemple en matériau conducteur ou semi-conducteur, l'adsorption et la désorption des espèces gazeuses provoquant une modification de la conductivité électrique de la zone sensible qui peut être détectée par les moyens de détection électrique adaptés.

Les moyens de chauffage de type à effet Joule peuvent être utilisés comme moyens de détection de type résistif.

Le capteur selon l'invention peut fonctionner soit un régime stationnaire, soit en régime transitoire ou dynamique.

Dans un état stationnaire, i.e. indépendant du temps, la concentration en analyte sur la zone sensible peut s'écrire en termes de fraction de sites actifs occupés par les espèces moléculaire (1 - $\theta$). Pour une adsorption sur une seule couche, la variation de la fraction en fonction du temps s'écrit :

$$\frac{d\theta}{dt} = k_{ads}(1 - \theta)c - k_{des}\theta \qquad \qquad 1)$$

Avec c la concentration de l'analyte dans le gaz, $k_{ads}$ la constante d'avancement de la réaction d'adsorption et $k_{des}$ la constante d'avancement de la réaction de désorption.

En supposant une surface homogène et que les atomes des sites adjacents n'interagissent pas.

On peut écrire dans un état stationnaire :

$$k_{ads}(1 - \theta_e)c - k_{des}\theta_e = 0$$
$$\theta_e = \frac{1}{1 + ck_{eq}} \qquad \qquad 2)$$
$$k_{eq} = \frac{k_{ads}}{k_{des}} = \frac{\theta_e}{(1 - \theta_e)c}$$

$K_{eq}$ étant la constant d'équilibre.

Dans le cas d'une adsorption multicouche, et en supposant un système limité de réactions, la constant d'équilibre $k_{eq}$ peut s'écrire:

$$k_{eq} = \frac{c_S}{c} = \frac{1}{c}\frac{\Delta m}{MSt} \approx \frac{1}{c}\frac{\Delta m}{MS} \qquad \qquad (3)$$

Avec $c_s$ la concentration du gaz dans la zone sensible en mol/m$^3$, $c$ la concentration de l'analyte dans le mélange gazeux en mol/m$^3$, $M$ la masse molaire (g/mol), $S$ la surface de la zone sensible en m$^2$ and $t$ (m) l'épaisseur de la zone sensible.

La connaissance de K$_{eq}$ permet de discriminer les différentes espèces gazeuses, par exemple une constante d'équilibre $k_{eq}$ élevée est caractéristique d'une affinité forte entre l'espèce gazeuse et la couche active.

Il est donc possible de distinguer les différentes espèces.

De même, dans un état transitoire ou dynamique; i.e. dans lequel on tient compte du temps dans la variation de la quantité d'espèce adsorbés et désorbées, l'évolution cinétique des adsorptions et désorptions dépend de la concentration en espèce gazeuses, des paramètres cinétiques et de la densité des sites d'adsorption disponibles. La réponse dans le temps de la zone sensible fournit des constantes qui peuvent être utilisés pour discriminer des espèces gazeuses en termes d'affinité entre les espèces et le couche de la zone sensible l'évolution cinétique est exprimée en termes de temps d'adsorption $\tau_{ads}$ et du temps nécessaire à la couche de la zone sensible pour être à nouveau fonctionnelle $\tau_{des}$:
On peut écrire :

$$\tau_{ads} \propto \frac{1}{k_{ads}c + k_{des}} \quad \text{and} \quad \tau_{des} \propto \frac{1}{k_{des}} \qquad 4)$$

De plus la vitesse de réaction dépend de la vitesse selon l'équation d'Arrhenius :

$$r = -\frac{\partial \sigma}{\partial t} = k_0 \sigma^n e^{-\frac{t}{\tau}} \qquad (5)$$

$$k_{eq} = \frac{k_{ads}}{k_{des}} = \alpha * exp\left[\frac{E_{des} - E_{ads}}{RT} t\right]$$

Avec r la vitesse de réaction en mol/cm$^3$ sec, $E$ l'énergie d'activation en kJ/mol, $\sigma^n$ le pourcentage de la surface qui interagit avec le gaz, n l'ordre du procédé et $k_0$ le facteur pré-exponentiel, ce dernier paramètre tient compte de la vitesse de variation de la température, de la concentration initiale adsorbée et l'énergie d'activation.

Il en résulte qu'avec les constantes $\tau_{ads}$ et $\tau_{des}$, $k_{eq}$ peut être utilisé comme facteur de discrimination.

Ainsi chaque espèce gazeuse ayant une affinité différente avec la couche de la zone sensible, une vitesse d'adsorption et une vitesse de désorption différentes, il est possible en appliquant des profils de température à la zone sensible de séparer les espèces gazeuses. Les moyens de contrôle sont alors programmés pour appliquer des profils de température adaptés à l'analyse d'espèces données. Les moyens de détection en mesurant une variation par exemple de fréquence de résonance de la partie suspendue ou de conductivité, permettent de déterminer quelles espèces ou familles d'espèces sont présentes dans le mélange gazeux et en quelle quantité.

Nous allons maintenant décrire des exemples de profils de température qui peuvent être appliquée à la zone sensible par les moyens de chauffage en fonction des ordres émis par les moyens de contrôle.

Nous décrirons tout d'abord des profils adaptés à un régime stationnaire.

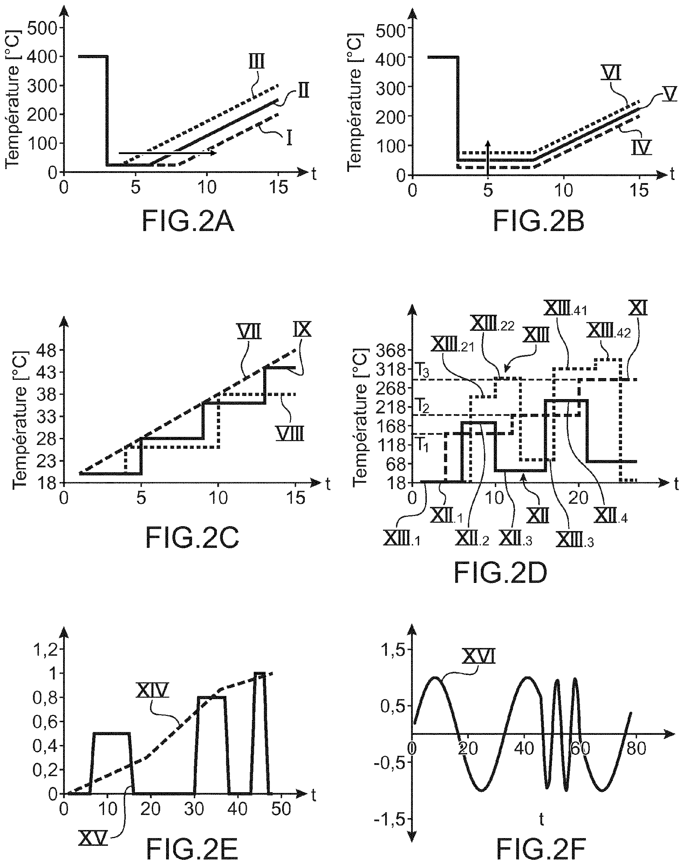

Les profils des figures 2A à 2C sont des profils adaptés à l'adsorption.

Sur la figure 2A, on peut voir différents profils de température en fonction du temps adaptés à une étape d'adsorption. On peut voir trois profils différents I, II, III qui présentent une zone à température constante pendant une durée déterminée, cette durée étant croissante du profil I au profil III. En faisant varier la durée de la phase à température constante, on augmente la quantité d'espèces adsorbées.

Sur la figure 2B, on peut voir trois profils de température IV, V, VI à température constante, les températures étant différentes, et les durées égales, la température augmentant du profil IV au profil VI.

Sur la figure 2C, on peut voir un profil VII imposant une température croissante linéairement avec le temps et deux profils VIII et IX imposant une augmentation de la température par palier. Toutes les espèces ne s'adsorbent pas nécessairement à la même température, on peut en imposant une augmentation de la température d'adsorption détecter l'adsorption des différentes espèces au fur et à mesure que la température augmente.

Le suivi de l'adsorption se fait par les moyens de détection qui détectent la variation de la quantité d'espèces adsorbés. Lors de l'adsorption, tout ou partie des espèces est adsorbées simultanément.

Sur la figure 2D, on peut voir un profil XI adapté à la désorption qui comporte des paliers de température croissante. A chaque palier une espèce ou une famille d'espèces est désorbée. Chaque désorption d'une espèces ou famille d'espèce provoque une variation de la grandeur physique de détection, cette variation est mesurée par les moyens de détection. Cette variation est à la fois relative à l'espèce ou famille d'espèces désorbées et à la quantité désorbée. Les valeurs de température des paliers sont choisies pour désorber chacun une espèce ou famille d'espèces adsorbées. Par exemple, supposons que le mélange gazeux comporte trois espèces A, B, C qui se sont adsorbées sur la zone sensible et que A se désorbe à une température T1, que B se désorbe à une température T2 et C se désorbe à une température T3 supérieure à T2. En appliquant le profil de désorption XI, les espèces A, B, C vont être désorbées séparément et les moyens de détection vont détecter chaque désorption.

Le profil XII est tel qu'il permet d'alterner des phases d'adsorption et de désorption. Par exemples, les portions XII.1, XII, 3 sont des isothermes permettant l'adsorption et les portions XII.2 et XII.4 sont des isothermes permettant la désorption.

En alternant des phases d'adsorption et de désorption on peut affiner la connaissance de l'espèce adsorbée. Par exemple, l'adsorption XII.1 peut se faire « à l'aveugle » et la désorption XII.2 également. Ensuite ayant détecté une désorption, la température de l'adsorption XII.3 est augmentée pour cibler davantage l'espèce. Ensuite dans l'exemple représenté, la température de désorption XII.4 est également augmentée. On peut envisager d'adapter le profil en fonction des mesures faites pas les moyens de détection afin de cibler au mieux le ou les espèces ou d'appliquer des profils déjà programmés. En effet, si rien n'est adsorbé en augmentant la température d'adsorption, c'est que cela signifie que cette température est au moins égale à celle de désorption. Les températures d'adsorption et de désorption sont donc à modifier.

Le profil XIII est adapté à l'analyse d'un mélange gazeux comportant au moins deux espèces ou deux familles d'espèces. Le profil comporte des phases de désorption comportant deux paliers, permettant donc deux désorptions successives à deux températures différentes.

On considère un mélange gazeux comportant trois espèces A, B, C.

Lors de la phase XIII.1 les trois espèces sont adsorbées.

Lors de la phase XIII.21, les espèces B et C sont désorbées et lors de la phase XIII.22 l'espèce A est désorbée.

Lors de la phase XIII.3, une nouvelle phase d'adsorption a lieu, seules les espèces B et C sont adsorbées.

Lors de la phase XIII.41 l'espèce B est désorbée et lors de la phase XIII.42 l'espèce C est désorbée.

Ainsi les trois espèces ont pu être séparées et la variation du paramètre physique relative à la désorption de chaque espèce a pu être mesurée. On peut alors identifier chaque espèce et la quantité adsorbée. On peut alors remonter à la concentration de chaque espèce dans le mélange gazeux.

Dans un régime dynamique, les temps ou vitesses d'absorption et de désorption peuvent être utilisés pour détecter les différentes espèces.

Sur la figure 2E, on peut voir deux exemples de profils de température adaptés à une analyse en régime dynamique lors duquel on observe à la fois de l'adsorption et la désorption. Le profil XIV comporte une série de rampes croissantes et le profil XV comporte des impulsions dont l'extremum croît à chaque impulsion, les impulsions pouvant avoir ou non des durées identiques ou différentes.

Sur la figure 2F, on peut voir un profil XVI en forme de signal sinusoïdal, l'amplitude du signal est constante dans le temps mais a une fréquence variable. Inversement, on pourrait prévoir un signale à amplitude variable et à fréquence constante. Par exemple un signal commandant les moyens de chauffage présentant plusieurs fréquences peut directement modifier la cinétique de réaction sur la zone sensible.

Dans les représentations des figures 2E et 2F la température en ordonnée est normalisée.

Le signal en régime dynamique ou transitoire peut combiner des signaux sinusoïdaux, des rampes des impulsions à différentes fréquence, le bruit. En effet le bruit d'une réponse en fréquence peut permettre de discriminer les espèces gazeuses dans un environnement.

Le fonctionnement du capteur en régime dynamique peut permettre de mieux détecter la désorption qui peut être très rapide. En effet, en faisant croître continûment la température on passe continûment d'une phase d'adsorption à une phase ou des phases de désorption successives. Dans le cas d'un mode de détection en régime stationnaire, par exemple en appliquant des profils en palier, le temps requis pour atteindre le palier, la désorption pour au moins une espèce peut être déjà terminée. Ainsi le fonctionnement en capteur en régime dynamique peut permettre de détecter des désorptions d'espèces spécifiques et de les identifier par rapport à la désorption d'autres espèces si la désorption de l'espèce recherché à une dynamique trop rapide ou une température associée trop proche de la température de désorption des espèces gazeuses présentes dans le mélange mais non recherchées. L'utilisation d'un fonctionnement statique ou dynamique est choisie en fonction des mélanges gazeux, des espèces gazeuses recherchées et de l'adéquation des espèces recherchées avec les caractéristiques physico-chimiques des couches sensibles retenues. Les deux modes de fonctionnement peuvent être utilisés pour augmenter la sélectivité du capteur, séparément ou en même temps.

Lorsque le mode de fonctionnement du capteur implique d'adsorber et désorber plusieurs fois tout ou partie des espèces gazeuses sur la même zone sensible du même capteur, comme c'est par exemple le cas des profils XII, XIII, XVI, on prévoit de maintenir le volume de gaz isolé de l'environnement externe au voisinage du capteur. Le capteur peut alors être intégré au sein d'un canal fluidique ou microfluidique qui est connectée à un système de circulation gazeuse et à un système de vannes en entrée et en sortie du canal. La fermeture des vannes permet de confiner le mélange gazeux à analyser au voisinage du capteur pendant un temps contrôlé pendant la durée de l'analyse. De manière très avantageuse, les vannes sont du type normalement fermées, elles sont donc actionnées pour permettre au mélange gazeux à analyser d'entrer dans le canal, ce qui permet de réduire la consommation électrique.

Dans le cas où plusieurs capteurs ayant la même couche sensible sont utilisés comme cela sera décrit ci-dessous, on prévoit également de maintenir le volume de gaz isolé de l'environnement externe au voisinage des capteurs.

Lorsqu'on met en oeuvre plusieurs capteurs on peut prévoir d'adsorber ou désorber plusieurs fois les espèces gazeuses séquentiellement sur les couches sensibles des capteurs, le confinement des capteurs peut être réalisé en les intégrant au sein d'une structure d'un canal fluidique ou microfluidique ce canal peut être réalisé en assemblant deux plaques structurées par des techniques de micro-fabrication classiques. Le canal peut avantageusement être connecté à un système de circulation gazeuse, par exemple une pompe, qui impose une direction au flux de gaz, ce qui permet de désorber et d'adsorber les espèces sur les différentes zones sensibles des différents capteurs selon un ordre spatial donné. En l'absence d'un système de circulation gazeuse, les espèces se déplacent d'un capteur à l'autre dans les deux sens du canal microfluidique et uniquement par diffusion.

Nous allons maintenant donner un exemple de mesure en régime stationnaire d'une mesure réalisée sur un mélange gazeux comportant au moins trois espèces A, B et C.

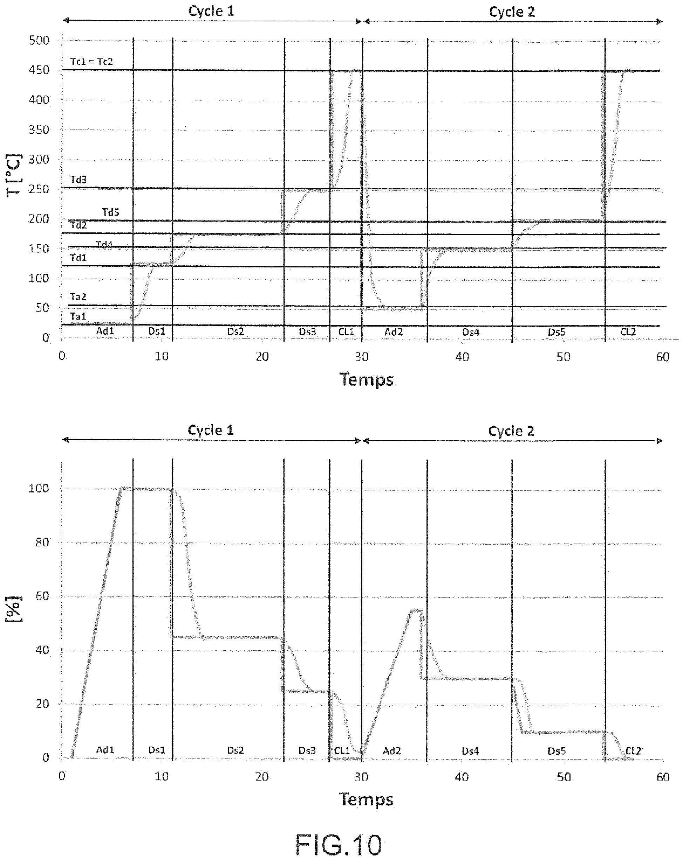

Le profil de température et le signal des moyens de détection en fonction du temps t sont représentés sur la figure 10.

Le profil de température comporte deux cycles, chaque cycle comportant une phase d'adsorption des espèces A, B et C et des phases de désorption successives.

En trait foncé, il s'agit de la consigne ou réponse idéale et en trait clair il s'agit de la réponse réelle.

Les phases d'adsorption sont désignées Ad1 et Ad2 et les phases de désorption sont désignées Ds1 à Ds6.

Le moyens de détection fournissent des signaux lors de chaque phase, ceux-ci sont désignés Sa1 et Sa2 pour les phases Ad1 et Ad2 et Sd1 à Sd6 pour Ds1 à Ds6.

Les signaux sont exprimés en pourcentages, le signal lors de la première phase d'adsorption est égal à 100%, lorsqu'une partie au moins des espèces est désorbée le signal est inférieure à 100%.

Les moyens de chauffage sont commandés par les moyens de contrôle pour appliquer lors de la phase Ad1 une température Ta1. Le signal Sa1 est composé sensiblement d'une fonction linéaire caractéristique d'une variation continue de la quantité d'espèce adsorbée. Dans l'exemple représenté, la valeur de la caractéristique physique augmente mais il sera compris qu'elle pourrait diminuer.

Lors de la phase Ds1 une température Td1 supérieure à Ta1 est appliquée à la zone sensible. Le signal Sd1 mesuré par les moyens de détection est stationnaire et est égal à la valeur du signal Sa1 à la fin de l'étape Ad1, ce qui signifie qu'aucun événement détectable pas les moyens de détection n'est survenu à Td1, notamment aucune désorption. Les espèces A, B et C se désorbent de la zone sensible pour une température supérieure à Td2.

Lors de la phase Ds2, une température Td2 supérieure à Td1 est appliquée. Le signal Sd2 est de l'ordre de 45%. Les espèces A et B se sont désorbées.

Lors de la phase Ds3, une température Td3 supérieure à Td2 est appliquée. Le signal Sd3 est de l'ordre de 25%. L'espèce C est désorbée. Le signal Sd3 n'est pas à 0% ce qui signifie que d'autres espèces différentes des espèces d'intérêt ont été adsorbées.

Lors d'une phase de nettoyage CL1, la température TC1 supérieure à Td3 est appliquée pour provoquer la désorption des espèces restantes, le signal Sc1 est à 0%. Le capteur est prêt pour une nouvelle mesure.

Lors du premier cycle, l'espèce C a pu être isolée, la différence entre les signaux Sd2 et Sd3 permet de déterminer l'espèce C et la quantité d'espèce C.

On réalise un deuxième cycle de mesure pour isoler A et B.

Lors d'un deuxième cycle de mesure, les moyens de chauffage sont commandés par les moyens de contrôle pour appliquer lors de la phase Ad2 une température Ta2 différente de Ta1 afin d'adsorber les espèces A et B mais pas l'espèce C. Le signal Sa2 est composé sensiblement d'une fonction linéaire caractérisant l'adsorption sur la zone sensible, le maximum de Sa2 est de l'ordre de 55%, inférieur à 100% car au moins l'espèce C n'est pas adsorbée ainsi que d'autres espèces sans intérêt pour la présente mesure.

Lors de la phase Ds4 une température Td4 supérieure à Ta2 est appliquée à la zone sensible. Le signal Sd4 mesuré par les moyens de détection est de l'ordre de 30% est stationnaire et est inférieur au signal Sa2, l'espèce A est désorbée.

Lors de la phase Ds5, une température Td5 supérieure à Td4 est appliquée. Le signal Sd5 est de l'ordre de 10%. L'espèce B est désorbée. 10% d'espèces sans intérêt ont été adsorbées.

Une phase de nettoyage CL2 a lieu à une température TC2 supérieure à Td5, le signal Sc2 est à 0%, dans l'exemple représentée TC1 = TC2. Le capteur est prêt pour une nouvelle mesure.

Lors de ce deuxième cycle, les espèces A et B ont été séparées et des signaux spécifiques pour ces deux espèces ont été obtenus permettant de déterminer la quantité de chacune des espèces.

**EP 3 185 011 B1**

Il sera compris que le nombre de cycles peut varier en fonction du nombre d'espèces d'intérêt à détecter. Les températures d'adsorption et de désorption sont choisies en fonction des espèces que l'on souhaite détecter.

Dans le cas d'un mode de fonctionnement dynamique, le profil de température et le signal des moyens de détection en fonction du temps t obtenus seraient similaires à ceux de la figure 10, mais on fait varier la fréquence et/ou l'amplitude de la fonction d'activation.

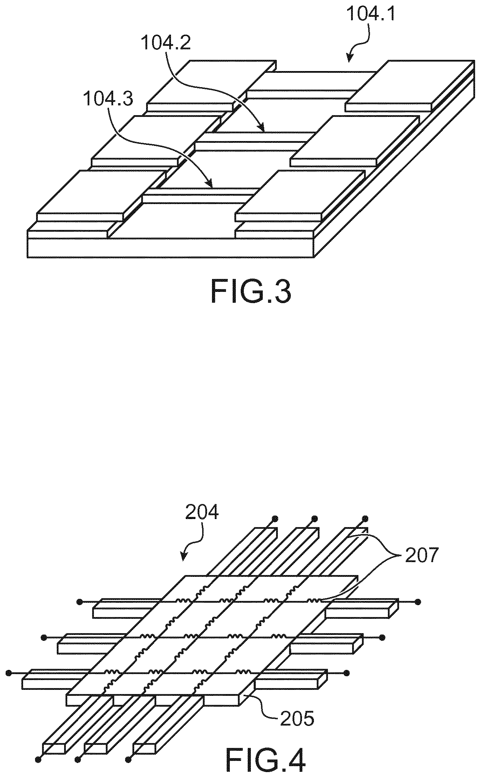

Sur la figure 3, on peut voir un autre exemple de réalisation d'un capteur selon l'invention comportant plusieurs parties suspendues 104.1, 104.2, 104.3 formées par des poutres suspendues et disposées parallèlement les unes par rapport aux autres. Les poutres présentent des sections différentes les unes des autres, de sorte que la surface de la zone sensible varie d'une poutre à l'autre. En variante, les poutres pourraient se croiser.

Il peut par exemple être prévu de chauffer chaque zone sensible à une température différente pour effectuer des mesures simultanément à des températures différentes, par exemple ce qui permet d'adsorber ou désorber certaines espèces sur une zone sensible et d'autres espèces sur d'autres zones sensibles.

Le capteur de la figure 3 peut être disposé dans un canal. Toutes les zones sensibles sont chauffées à la même température, chaque zone sensible adsorbe en même proportion les mêmes espèces, en revanche la quantité d'espèces adsorbés est plus grande pour les zones sensibles de plus grande surface. Par comparaison on peut distinguer les mesures relatives aux espèces adsorbées et désorbées et corriger différents défauts, tels que la dérive. En réalisant la comparaison du signal de 104.1 et 104.3 et en observant la différence des deux signaux on peut supprimer les variations du signal (bruit, dérive) qui sont liés à des effets parasites de l'absorption/désorption (température, pression) et ainsi n'observer que la composante du signal qui est lié à l'adsorption/désorption d'espèces gazeuses sur la partie de la couche sensible de 104.1 qui est en excès par rapport à la couche sensible de 104.3. La réponse fournie par la couche sensible 104.1 devrait être plus forte que celle fournie par la couche sensible 104.3, mais devrait être également plus affecté par des artefacts. En combinant les deux réponses, on peut réduire les interférences des autres gaz sur l'installation.

Sur la figure 4, on peut voir un autre exemple de capteur dans lequel la partie suspendue 204 comporte une membrane 205 sur laquelle est réalisé un réseau de conducteurs électriques 207 couvrant la membrane. Dans l'exemple représenté les conducteurs électriques 207 forment un quadrillage. En supposant que la zone sensible recouvre toute la membrane, on peut par exemple mesurer la résistance d'un conducteur ou de plusieurs conducteurs couplés ce qui permet de détecter les phases d'adsorption et de désorption à différents endroits de la zone sensible. On peut alors travailler à différentes températures simultanément et par exemple en déduire des informations sur le bruit de mesure et corriger les mesures en conséquence.

De manière très avantageuse, les moyens de commande sont programmés de sorte que, dans le cas où la variation de la propriété physique mesurée pour la détection est inférieure à une valeur seuil lors d'une phase d'adsorption, on considère que la ou les étapes de désorption n'ont pas à avoir lieu car l'environnement ne contient pas ou pas suffisamment de l'espèce ou des espèces d'intérêt. Ce mode de fonctionnement permet de réduire la consommation d'énergie du capteur. Dans le cas où le capteur est utilisé pour détecter la présence de polluants ou produits dangereux, cette détection lors de l'adsorption en dessous de la valeur seuil peut permettre de conclure que l'environnement est dépourvu de ces espèces et est donc sûr. De manière très avantageuse, une étape de nettoyage a lieu pour remettre la zone sensible dans un état de référence.

Comme cela a déjà été mentionné, les moyens de contrôle peuvent être de type adaptatif et donc modifiés les signaux de contrôle aux moyens de chauffage en fonction des signaux de sortie délivrés par les moyens de détection. Par exemple, le profil de température appliqué en premier comporte une adsorption isotherme et une désorption isotherme et les profils suivants peuvent par exemple comporter une adsorption isotherme et des désorptions à différentes isothermes ou à température croissante afin de cibler au mieux les espèces et les séparer. Cette adaptabilité peut être particulièrement intéressante lorsque que l'on ne connaît pas le nombre d'espèces d'intérêt contenu dans l'environnement ou le mélange gazeux.

Les moyens de contrôle peuvent en outre être programmés pour effectuer une étape de nettoyage de la zone sensible en appliquant une température suffisamment élevée pour désorber toutes les espèces d'intérêt ou non. L'efficacité du nettoyage est vérifiée en analysant les signaux des moyens de détection qui doivent correspondre à une zone sensible libre de toutes espèces ou au moins correspondre à une zone sensible dans un état de référence connu.

Dans un exemple avantageux, la mesure du bruit et l'analyse de la dépendance en température des spectres de bruit électriques à des conditions de travail sélectionnées peuvent être utilisées pour détecter et discriminer les espèces gazeuses.

Comme indiqué ci-dessus, le capteur peut être disposé dans un canal ou microcanal. Le canal peut présenter une section variable, par exemple une section qui diminue dans la direction de l'écoulement, permettant de favoriser la séparation des espèces.

Par exemple dans le cas du capteur de la figure 3 ou plus généralement d'un capteur avec une poutre suspendue par des extrémités ou en porte-à-faux, la ou les poutres sont disposées de préférence orthogonalement à la direction du flux d gaz.

Dans un exemple avantageux, des moyens de chauffage supplémentaires, par exemple des résistances électriques, peuvent être intégrés au microcanal de sorte à générer un gradient thermique dans le canal et autour du capteur et/ou à contrôler les propriétés de l'environnement du capteur.

Dans un autre exemple de réalisation, le capteur peut être associé à une pompe, plus particulièrement une micropompe. La micropompe, en générant un écoulement dans le canal peut permettre de nettoyer la ou les zones sensibles par exemple après une phase de nettoyage et de désorption de toutes les espèces adsorbées. La pompe sert également à établir un flux de gaz contrôlé au voisinage des capteurs ce qui peut permettre de déplacer une espèce désorbée à partir d'une zone sensible vers un autre capteur, pour être absorbée sur une autre zone sensible.

La pompe ou micropompe peut être une pompe de type Knudsen qui est une pompe bidirectionnelle actionnée thermiquement. Cette pompe présente l'avantage de ne pas avoir de partie mobile. Une telle pompe en matériau poreux comprenant des pores de 100 nm peut générer un débit de 0,74 sccm. Une telle pompe est par exemple décrite dans « Knudsen pump driven by a thermoelectric material », Kunal Pharas and Shamus McNamara, Published 29 November 2010 • 2010 IOP Publishing Ltd - Journal of Micromechanics and Microengineering, Volume 20, Number 12

Comme décrit ci-dessus, on peut réaliser des capteurs avec plusieurs parties suspendues dont la température est commandée par les mêmes moyens de contrôle et on peut réaliser des systèmes de détection mettant en oeuvre plusieurs capteurs selon l'invention, chaque capteur comporte ses propres moyens de commande.

Par exemple, dans un canal plusieurs capteurs peuvent être intégrés de manière similaire à celle représentée sur la figure 3.

Les capteurs peuvent avoir des caractéristiques différentes par exemple en termes de tailles, de couche sensible... Ils peuvent ainsi être distingués les uns des autres. En outre, ils peuvent présenter des moyens de chauffage différents les uns des autres. En outre, la commande des moyens de chauffage peut différer d'un capteur à l'autre, appliquant ainsi des profils de température différents. On peut par exemple envisager que certains capteurs adsorbent certaines espèces et d'autres capteurs adsorbent d'autres espèces.

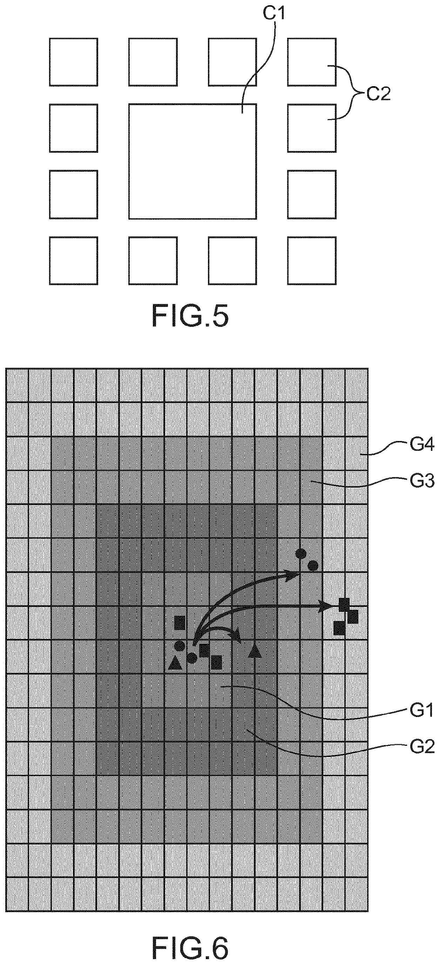

Sur la figure 5, on peut voir un exemple d'un dispositif comportant un premier capteur C1 selon l'invention comportant une zone sensible apte à adsorber et désorber des espèces et une pluralité de seconds capteurs C2 selon l'invention disposés autour du premier capteur C1, comportant une zone sensible aptes à adsorber les espèces désorbées par le premier capteur. Les seconds capteurs C2 permettent d'analyser les espèces désorbées par le premier capteur. Cette combinaison est particulièrement intéressante car le capteur selon l'invention comportant des moyens de chauffage commandés selon des profils de donnés peut séparer les espèces ou familles d'espèces adsorbées et les second capteurs C2 analysent les espèces séparées. Dans cet exemple, le premier capteur sert de séparateur et de détecteur d'espèces ou de familles d'espèces. En variante, les seconds capteurs C2 pourraient être des capteurs de gaz de l'état de la technique.

Les informations fournies par les capteurs C1 et C2 sont complémentaires et sont utilisés de manière complémentaire pour déduire une information sur l'espèce gazeuse à détecter

Sur la figure 6, on peut voir un autre exemple de réalisation d'un dispositif comportant une pluralité de capteurs selon l'invention répartis en groupes, quatre dans l'exemple représenté, disposés de manière concentrique. Le groupe G1 forme un pavé de capteurs au centre du système, le groupe G2 entoure le groupe G1, qui est entouré par le groupe G3, qui est lui-même entouré par le groupe G4.

Chaque groupe est à une température donnée de sorte à établir un gradient de température entre le groupe G1 et le groupe G4.

Le groupe G1 adsorbe toutes les espèces puis les désorbe, ces espèces sont ensuite sélectivement adsorbées par l'un des capteurs de l'un des groupes en fonction de sa température d'adsorption. Le gradient de température a également pour effet de provoquer le déplacement des espèces désorbées. On peut également prévoir que les zones sensibles soient différentes, certaines espèces s'adsorbant sur les zones actives d'un groupe et pas des autres groupes. Les espèces différentes sont schématisées par les formes en carré, triangle et rond.

La température des capteurs du groupe G1 peut être commandée pour assurer une séparation des espèces ou familles d'espèce.

On peut prévoir de manière très avantageuse d'enregistrer les instants d'adsorption d'espèces par les capteurs de chaque groupe, ce qui permet d'obtenir des informations sur les propriétés d'adsorption et de désorption stationnaire et dynamique et également de connaître la vitesse de déplacement des espèces.

En outre le gradient thermique imposé permet de maîtriser la direction d'écoulement du flux de gaz.

L'amplitude, la fréquence et la modulation de phase des signaux de température peuvent être utilisées pour étudier leurs effets sur les processus d'adsorption/désorption combinés. En effet, les processus de désorption/adsorption dépendent du temps; l'amplitude, la fréquence et la modulation sont utilisées pour discriminer les différentes espèces gazeuses et pour augmenter la sélectivité du capteur. Par exemple différentes zone pourraient être activées avec un signal d'actionnement particulier, par exemple pour réaliser un réseau de plusieurs capteurs ciblant des espèces gazeuses différentes en vue de réaliser un capteur polyvalent pour plusieurs espèces.

On peut envisager d'avoir plusieurs capteurs disposés les uns à côtés des autres avec des couches sensibles différentes.

On peut prévoir de réaliser un système MEMS et/ou NEMS comportant plusieurs zones sensibles, chacune sur une partie suspendue distinctes ou sur une seule partie suspendue, les zones sensibles étant empilées.

Les moyens de contrôle peuvent également être programmés pour appliquer tous les capteurs de tous les groupes une étape de nettoyage thermique, i.e. de désorption de toutes les espèces afin de remettre les capteurs dans un état de référence.

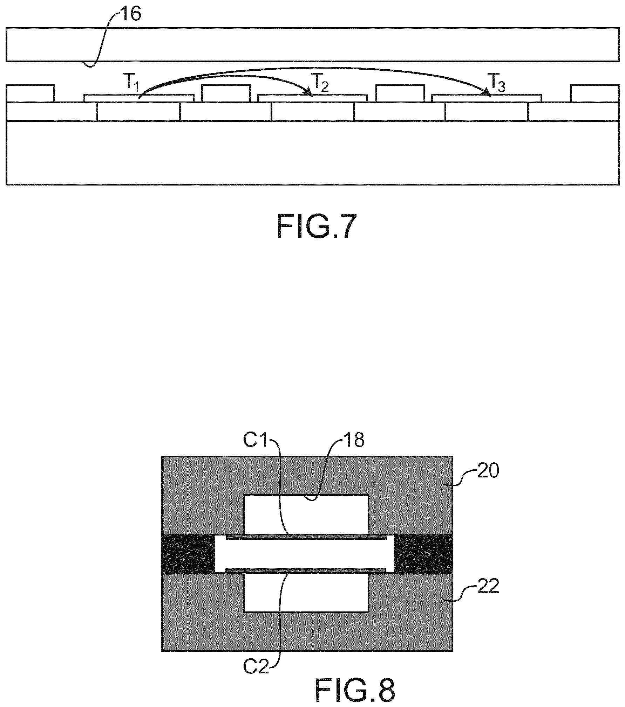

Sur la figure 7, on peut voir un système de détection comportant un canal 16 dans lequel sont disposés plusieurs capteurs C1, C2, C3 disposés les uns à la suite des autres dans l'axe du canal, les zones sensibles n'étant pas en contact les uns des autres. En appliquant un gradient de température dans le canal par exemple par des moyens de chauffage intégrés au canal ou par les moyens de chauffage de chacune des capteurs, on peut établir un écoulement du gaz dans le canal, le gaz entrant en contact avec les capteurs successifs.

Sur la figure 8, on peut voir deux capteurs disposés dans un canal 18 sensiblement dans le même plan orthogonal à l'axe du canal. Le canal est délimité par deux substrats 20, 22 solidarisés l'un à l'autre de manière étanche. Une zone sensible d'un capteur est suspendue à un substrat et la zone sensible de l'autre capteur est suspendue à l'autre substrat.

Les capteurs peuvent être disposés les uns par rapport aux autres pour améliorer les performances de détection, par exemple en termes de sélectivité et sensibilité, et/ou pour corriger des erreurs de mesure, telles que la dérive.

Selon un exemple, les capteurs selon l'invention peuvent être disposés et connectés de sorte à permettre une mesure différentielle. Par exemple un premier capteur est disposé dans l'environnement gazeux à analyser et le second capteur est disposé dans un environnement contrôlé ou est tel qu'il n'est pas sensible aux mêmes gaz que le premier capteur. Les mesures peuvent alors être soustraites et permettent de supprimer des dérives dues à des variations dans les conditions de mesure.

Selon un autre exemple, les capteurs peuvent être disposés et connectés de sorte à permettre une mesure comparative. Par exemple, on utilise plusieurs capteurs ayant les mêmes zones sensibles. Par exemple on utilise trois capteurs alignés dans un canal fluidique, le capteur amont réalise une mesure initiale du mélange gazeux, le capteur central adsorbe les espèces gazeuses d'intérêt et le capteur aval, qui est identique au capteur amont, réalise une mesure sur le mélange gazeux. Les signaux du capteur aval sont comparés aux signaux du capteur amont et on peut détecter une différence ou non dans le mélange gazeux résultant de l'adsorption par le capteur amont.

Un système de détection selon l'invention peut également associer un ou des capteurs selon l'invention et un ou des capteurs d'un ou d'autres types, les signaux fournis par les capteurs de types différents permettant d'améliorer les performances de détection du système.

Le système comporte un capteur selon l'invention comportant une partie suspendue 304 recouverte d'une couche sensible 306 destinée à être mis en vibration, et un actionneur électrostatique 324 destiné à appliquer un champ électrique à la partie suspendue 324 pour la mettre en vibration. L'actionneur comporte trois électrodes, une 326 au centre et deux 328, 330 de part et d'autre de l'électrode centrale 326. L'application d'une différence de potentiel entre l'électrode centrale 326 et l'une 328, 330 des autres électrodes génère un champ électrique qui met en vibration la partie suspendue 304. La partie suspendue 304 est disposée de sorte à subir le champ électrique. Dans l'exemple représenté, la partie suspendue 304 est alignée avec l'électrode centrale 326. La mise en oeuvre de trois électrodes permet d'appliquer un champ électrique non uniforme sur la partie suspendue. Lorsque la zone sensible disposée sur la partie suspendue adsorbe ou désorbe des espèces, la fréquence du signal de sortie varie, l'amplitude et la déviation de la valeur moyenne par rapport à zéro du signal de sortie étant fixés par les électrodes de l'actionneur.

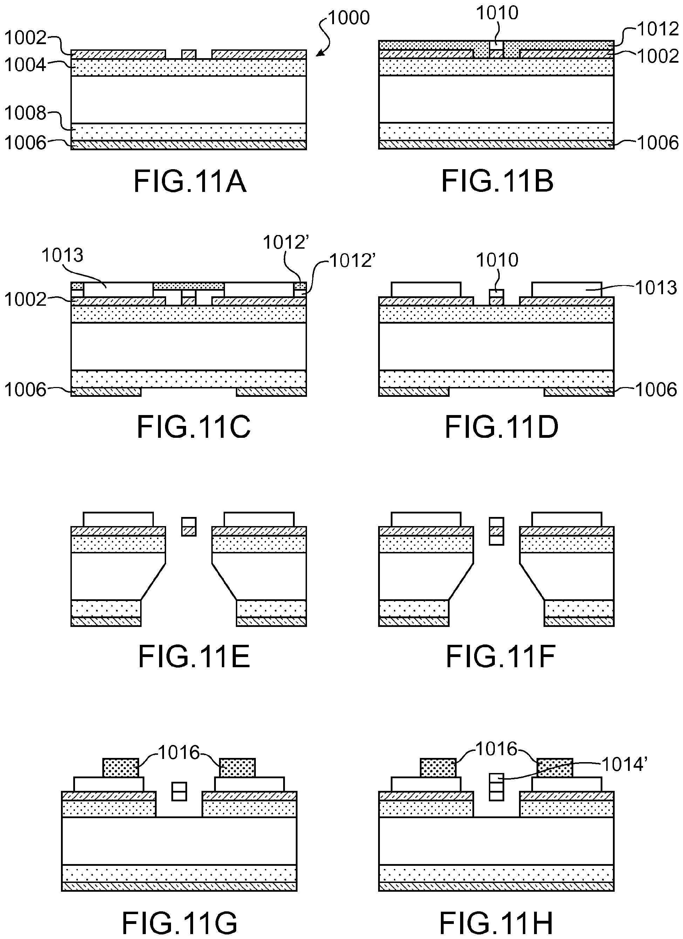

Un exemple de procédé de fabrication d'un capteur selon l'invention va maintenant être décrit à l'aide des figures 11A à 11H.

Lors d'une première étape, on utilise par exemple un substrat 1000 comportant en face avant une couche de silicium 1002 déposée sur une sacrificielle par exemple une couche d'oxyde de silicium ou de nitrate de silicium 1004 et en face arrière une couche de silicium 1006 déposée sur une couche d'oxyde de silicium ou de nitrate de silicium 1008. En variante un substrat silicium sur isolant ou SOI (Silicon on insulator en terminologie anglo-saxonne) pourrait être utilisé.

La couche de silicium 1002 est structurée pour former les éléments du MEMS et/ou NEMS, tels que la partie suspendue, les moyens d'actionnement par exemple les électrodes dans le cas d'un capteur résonant... la couche 1002 est par exemple structurée par dépôt d'une résine et gravure au moyen d'acide fluorhydrique, ou gravure KOH ou par gravure ionique réactive (Rear Ionic Etching en terminologie anglo-saxonne).

L'élément ainsi obtenu est représenté sur la figure 11A.

Lors d'une étape suivante, on réalise les moyens de chauffage sur la partie suspendue pour cela une couche conductrice électrique 1010, par exemple en métal, est formée sur la couche structurée 1002 par exemple par dépôt, cette couche 1010 est destinée à former les moyens de chauffage. Préalablement une couche de résine 1012 est formée sur la couche 1002 et est gravée pour accéder uniquement la face avant de la partie suspendue, ou une partie de celle-ci. Il peut être prévu de former une couche protectrice pour séparer les moyens de chauffage de la couche sensible.

L'élément ainsi obtenu est représenté sur la figure 11B.

Lors d'une étape suivante, une couche de résine 1012' est déposée et est gravée pour accéder à la couche 1002 afin de délimiter les zones de dépôt du métal en vue de la réalisation des contacts ou plots d'interconnexion. Ensuite, les contacts ou plots d'interconnexion sont réalisés en formant une couche de métal 1013 sur la couche de résinée 1012' et les zones gravées.

On grave également couche de silicium 1006 en face arrière.

L'élément ainsi obtenu est représenté sur la figure 11C.

Lord d'une étape suivante, la couche 1012 est retirée.

L'élément ainsi obtenu est représenté sur la figure 11D.

Lors d'une étape suivante, la partie suspendue est libérée par exemple par gravure humide au moyen de HF ou BOE ou par gravure sèche. Les oxydes et les métaux utilisés sont choisis pour être résistants à cette gravure. La libération se fait par gravure en face arrière à travers tout l'empilement à partir de la couche d'oxyde 1008.

L'élément ainsi obtenu est représenté sur la figure 11E.

Lors d'une étape suivante, on forme la zone sensible par exemple en déposant une couche sensible 1014 directement sur tout ou partie de la partie suspendue.

La couche sensible est par exemple réalisée par dépôt d'espèces chimiques en solution, par exemple par technique de Langmuir-Blodgett, par enduction centrifuge ou par pulvérisation.

En variante, elle peut être formée par un procédé de dépôt en phase gazeuse, par exemple par dépôt chimique en phase vapeur, par dépôt en phase vapeur ou par dépôt en phase vapeur moléculaire.

La technique de dépôt de la zone sensible est telle que celle-ci est formée d'un film mince d'épaisseur contrôlée et homogène, par exemple de l'ordre quelques dizaines à quelques centaines de nm.

L'élément ainsi obtenu est représenté sur la figure 11F. Dans cet exemple la couche sensible est formée sur l'une des faces de la partie suspendue, mais elle pourrait être formée sur les deux faces de la partie suspendue.

Selon une variante, on réalise une couche d'oxyde supplémentaire 1016 sur la structure du MEMS et/ou NEMS forme une couche de planarisation par exemple dans le cas d'un montage dans un canal fluidique. En outre elle peut servir à protéger le capteur des dommages mécaniques et des solutions utilisées dans la suite du procédé.

La partie suspendue est libérée par exemple par gravure humide au moyen de HF ou BOE ou par gravure sèche. Les oxydes et les métaux utilisés sont choisis pour être résistants à cette gravure. Dans cette variante, la libération est obtenue en ne gravant que la coude d'oxyde 1004.

L'élément ainsi obtenu est représenté sur la figure 11G.

Lors d'une étape suivante, la zone sensible 1014' est formée sur la partie suspendue comme décrit ci-dessus. Dans cette variante, la zone sensible 1014'est formée sur le métal formant les moyens de chauffage.

L'élément ainsi obtenu est représenté sur la figure 11H.

La zone sensible peut être formée avant ou après libération de la partie suspendue.

De préférence lorsque la réalisation de la zone sensible requiert un dépôt de résine, la partie suspendue est libérée après la réalisation de la zone sensible pour éviter une rupture par exemple des poutres de suspension.

Dans le cas où la zone sensible est formée par pulvérisation, elle peut être réalisée après la libération de la partie suspendue.

Dans le cas où l'on souhaite intégrer le capteur dans un canal fluidique, on réaliser un deuxième substrat dans lequel le canal est gravé. Les deux substrats sont ensuite assemblés de manière étanche par exemple en utilisant la couche 1016 comme zone d'interface. L'assemblage étanche est par exemple réalisé par une colle qui n'interagit pas avec les espèces gazeuses ou plus généralement une colle permettant d'assembler les substrats mais qui ne soit pas contaminante vis-à-vis du milieu à analyser, par exemple de type Ordyl®.

Le capteur selon la présente invention peut être utilisé dans un très grand nombre d'applications. Par exemple il peut être utilisé comme capteur d'humidité, comme capteur chimique ou biologique.

Il peut servir pour réaliser de la spectroscopie de masse, de la spectroscopie par désorption thermique.

Ce capteur peut être utilisé pour vérifier la qualité de l'air intérieur.

Le capteur étant de fonctionnement sûr, nécessitant peu d'entretien et présentant une faible consommation énergétique, il peut être intégré dans des dispositifs électroniques portables tels que des téléphones ou des tablettes.

Le capteur peut également être facilement intégré dans ou à la suite une microcolonne de chromatographie en phase gazeuse.

**Revendications**

1. Capteur de gaz microélectromécanique et/ou nanoélectromécanique pour l'analyse d'un mélange d'au moins deux espèces gazeuses ou familles d'espèces gazeuses à détecter comportant :

- une partie fixe (2),
- au moins une partie suspendue (4) par rapport à la partie fixe (2),
- au moins une zone sensible(6) portée par la partie suspendue (4), ladite zone sensible (4) étant apte à adsorber/absorber et à désorber des espèces gazeuses ou familles d'espèces gazeuses,
- des moyens de chauffage (8) au moins de la zone sensible (6),
- des moyens de détection (10) de l'adsorption/absorption et de la désorption des espèces gazeuses ou familles d'espèces gazeuse sur la zone sensible,
- des moyens de contrôle des moyens de chauffage (8),

**caractérisé en ce que** les moyens de détection et les moyens de contrôle sont configurés pour mettre en oeuvre au moins un cycle comprenant les étapes :

a) application à la au moins une zone sensible d'une première température pour provoquer une adsorption/absorption de toutes les espèces gazeuses à détecter,
b) mesure d'un signal de détection de l'adsorption/absorption,
c) puis, application d'une deuxième température pour provoquer la désorption d'une partie des espèces gazeuses,
d) mesure d'un signal de détection de ladite désorption,
e) répétition des étapes a), b), c) et d) avec des températures d'adsorption/absorption différente de celle de l'étape a) précédente, et jusqu'à ce que toutes les espèces d'intérêt soient séparées.

2. Capteur de gaz selon la revendication 1, comportant des moyens de mesure de la température de la zone sensible (6).

3. Capteur de gaz selon la revendication 1 ou 2, dans lequel la partie suspendue (4) est mobile et les moyens de détection comportent des moyens d'actionnement pour mettre en mouvement la partie suspendue et des moyens pour mesurer le déplacement de la partie suspendue , ou , dans lequel la partie suspendue (4) est au moins en partie conductrice électrique et les moyens de détection mesurent une variation de conductivité de la partie suspendue.

4. Capteur de gaz selon l'une des revendications 1 à 3, dans lequel les moyens de chauffage sont des moyens de chauffage à effet Joule, avantageusement la partie suspendue étant au moins en partie conductrice électrique et est destinée à s'échauffer par effet Joule par circulation d'un courant.

5. Capteur de gaz selon les revendications 3 et 4, dans lequel les moyens de détection et les moyens de chauffage sont confondus.

6. Capteur de gaz selon l'une des revendications 1 à 5, comportant une base de données comportant des profils de température prédéfinis.

7. Capteur de gaz selon l'une des revendications 1 à 6, comportant plusieurs partie suspendues comportant des zones sensibles de surface différentes et/ou ayant des propriétés d'adsorption/absorption et désorption différentes les unes des autres, et/ou dans lequel les moyens de chauffage sont tels qu'ils peuvent chauffer différentes zones de la partie mobile à des températures différentes simultanément.

8. Système microélectromécanique et/ou nanoélectromécanique de détection de gaz comportant au moins un premier capteur de gaz selon l'une des revendications 1 à 7 et au moins un deuxième capteur de gaz et des moyens de comparaison et de traitement des signaux issus des moyens de détection de chacun desdits capteurs, les capteurs comportant avantageusement des zones sensibles différentes.

9. Système de détection de gaz selon la revendication 8, dans lequel le deuxième capteur de gaz est un capteur de gaz selon l'une des revendications 1 à 7 le premier capteur de gaz étant disposé dans un environnement de référence et le deuxième capteur étant destiné à être en contact avec un mélange gazeux.

10. Système de détection de gaz selon la revendication 8, dans lequel le premier capteur de gaz et le deuxième capteur de gaz sont destinés à être disposés dans un même mélange gazeux, au moins une zone sensible du premier capteur de gaz étant apte à adsorber/absorber et à désorber une première espèce gazeuse ou première famille d'espèce gazeuse à détecter et le deuxième capteur de gaz étant un capteur de gaz selon l'une des revendications 1 à 10, au moins une zone sensible du deuxième capteur étant apte à adsorber/absorber et à désorber une deuxième espèce gazeuse ou deuxième famille d'espèce gazeuse à détecter.

11. Système de détection selon la revendication 8, comportant plusieurs deuxièmes capteurs disposés autour du premier capteur, les moyens de contrôle des deuxièmes capteurs étant tels que les deuxièmes capteurs adsorbent/absorbent les espèces gazeuses ou familles d'espèces désorbées par le premier capteur.

12. Système de détection selon l'une des revendications 8 à 11, comportant un premier capteur et un deuxième capteur disposé en amont du premier capteur et un autre deuxième capteur disposé en aval du premier capteur et des moyens de comparaison des signaux fournies par les moyens de détection des deux deuxièmes capteurs.

13. Système de détection selon l'une des revendications 10 à 12, comportant plusieurs premiers capteurs et dans lequel les deuxièmes capteurs sont répartis en plusieurs groupes agencés de manière concentrique autour des premiers capteurs, les moyens de contrôle des deuxièmes capteurs étant tels qu'ils contrôlent les moyens de chauffage de sorte que les zones sensibles de chaque groupe soient à une température différente de celles des autres groupes.

14. Système de détection selon l'un des revendications 8 à 13, comportant un canal dans lequel est ou sont montés les capteurs, et comportant avantageusement une pompe connectée au canal.

15. Procédé d'analyse d'un mélange gazeux comportant au moins deux espèces gazeuses ou au moins deux familles d'espèces gazeuses mettant en oeuvre un capteur selon l'une des revendications 1 à 7 ou un système de détection selon l'une des revendications 8 à 14, comportant au moins un cycle comprenant les étapes :

a) application à au moins une zone sensible d'une première température pour provoquer une adsorption/absorption de toutes les espèces gazeuses à détecter,
b) mesure d'un signal de détection de l'adsorption/absorption,
c) puis, application d'une deuxième température pour provoquer la désorption d'une partie des espèces gazeuses,
d) mesure d'un signal de détection de ladite désorption,
e) répétition des étapes a) ; b), c) et d) avec des températures d'adsorption/absorption différente de celle de l'étape a) précédente et jusqu'à ce que toutes les espèces d'intérêt soient séparées.

16. Procédé d'analyse selon la revendication 15, dans lequel les conditions d'adsorption/absorption et de désorption du cycle suivant sont déterminées en fonction des signaux obtenus à l'étape d) et/ou dans lequel les conditions de désorption de l'étape c) sont déterminées en fonction des signaux obtenus à l'étape b).

17. Procédé d'analyse selon la revendication 15 ou 16, comportant au moins préalablement à l'étape a), au moins une étape de nettoyage par application d'une température provoquant une désorption telle que la zone sensible se retrouve dans l'état de référence.

18. Procédé selon l'une des revendications 15 à 17, dans lequel lorsque la valeur du signal de détection à l'étape b) est inférieure à une valeur seuil, le procédé d'analyse est interrompu.

**Patentansprüche**

1. Mikroelektromechanischer und/oder nanoelektromechanischer Gassensor für die Analyse einer Mischung von wenigstens zwei zu detektierenden Gassorten oder Familien von Gassorten, umfassend:

- ein festes Teil (2),
- wenigstens ein bezüglich des festen Teils (2) aufgehängtes Teil (4),
- wenigstens eine empfindliche Zone (6), die durch das aufgehängte Teil (4) getragen wird, wobei die empfindliche Zone (6) dazu ausgelegt ist, Gassorten oder Familien von Gassorten zu adsorbieren/absorbieren und zu desorbieren,
- Mittel (8) zum Heizen wenigstens der empfindlichen Zone (6),
- Mittel (10) zur Detektion der Adsorption/Absorption und der Desorption der Gassorten oder Familien von Gassorten auf der empfindlichen Zone,
- Mittel zur Steuerung der Heizmittel (8),

**dadurch gekennzeichnet, dass** die Detektionsmittel und die Steuerungsmittel dazu ausgelegt sind, wenigstens einen Zyklus auszuführen, der die folgenden Schritte umfasst:

a) Anlegen einer ersten Temperatur an die wenigstens eine empfindliche Zone zum Bewirken einer Adsorption/Absorption von allen zu detektierenden Gassorten,
b) Messen eines Signals zur Detektion der Adsorption/Absorption,
c) dann Anlegen einer zweiten Temperatur zum Bewirken der Desorption eines Teils der Gassorten,
d) Messen eines Signals zur Detektion der Desorption,
e) Wiederholen der Schritte a), b), c) und d) mit Adsorptions-/ Absorptionstemperaturen, die verschieden sind von jener des vorhergehenden Schritts a), und bis alle interessierenden Sorten getrennt sind.

2. Gassensor nach Anspruch 1, umfassend Mittel zur Messung der Temperatur der empfindlichen Zone (6).

3. Gassensor nach Anspruch 1 oder 2, bei dem das aufgehängte Teil (4) beweglich ist, und die Detektionsmittel Betätigungsmittel umfassen, um das aufgehängte Teil in Bewegung zu versetzen, sowie Mittel zum Messen der Verlagerung des aufgehängten Teils, oder bei dem das aufgehängte Teil (4) wenigstens teilweise elektrisch leitend ist und die Detektionsmittel eine Variation der Leitfähigkeit des aufgehängten Teils messen.

4. Gassensor nach einem der Ansprüche 1 bis 3, bei dem die Heizmittel Jouleeffekt-Heizmittel sind, wobei vorzugsweise das aufgehängte Teil wenigstens teilweise elektrisch leitend ist und dazu ausgelegt ist, sich mittels Jouleeffekt durch Zirkulation eines Stroms zu erhitzen.

5. Gassensor nach den Ansprüche 3 und 4, bei dem die Detektionsmittel und die Heizmittel zusammengelegt sind.

6. Gassensor nach einem der Ansprüche 1 bis 5, umfassend eine Datenbank, die vordefinierte Temperaturprofile umfasst.

7. Gassensor nach einem der Ansprüche 1 bis 6, umfassend mehrere aufgehängte Teile, die verschiedene empfindliche Oberflächenzonen umfassen und/oder voneinander verschiedene Adsorptions-/Absorptions- und Desorptionseigenschaften haben, und/oder bei dem die Heizmittel derart ausgelegt sind, dass sie verschiedene Zonen des beweglichen Teils gleichzeitig auf verschiedene Temperaturen heizen können.

8. Mikroelektromechanischer und/oder nanoelektromechanischer System zur Detektion von Gas, umfassend wenigstens einen ersten Gassensor nach einem der Ansprüche 1 bis 7 und wenigstens einen zweiten Gassensor, sowie Mittel zum Vergleichen und zum Verarbeiten der Signale, die von den Detektionsmitteln jedes der Sensoren stammen, wobei die Sensoren vorzugsweise verschiedene empfindliche Zonen umfassen.

9. System zur Detektion von Gas nach Anspruch 8, bei dem der zweite Gassensor ein Gassensor nach einem der Ansprüche 1 bis 7 ist, wobei der erste Gassensor in einer Referenzumgebung angeordnet ist, und der zweite Sensor dazu ausgelegt ist, in Kontakt mit einer Gasmischung zu sein.

10. System zur Detektion von Gas nach Anspruch 8, bei dem der erste Gassensor und der zweite Gassensor dazu ausgelegt sind, in ein und derselben Gasmischung angeordnet zu sein, wobei wenigstens eine empfindliche Zone des ersten Gassensors dazu ausgelegt ist, eine erste zu detektierende Gassorte oder erste Familie von Gassorten zu adsorbieren/aborbieren und zu desorbieren, und bei dem der zweite Gassensor ein Gassensor nach einem der Ansprüche 1 bis 10 ist, wobei wenigstens eine empfindliche Zone des zweiten Gassensors dazu ausgelegt ist, eine zweite zu detektierende Gassorte oder zweite Familie von Gassorten zu adorbieren/absorbieren und zu desorbieren.

11. System zur Detektion nach Anspruch 8, umfassend mehrere zweite Sensoren, die um den ersten Sensor herum angeordnet sind, wobei die Steuermittel der zweiten Sensoren derart ausgelegt sind, dass die zweiten Sensoren die Gassorten oder Familien von Sorten adsorbieren/absorbieren, die durch den ersten Sensor desorbiert werden.

12. System zur Detektion nach einem der Ansprüche 8 bis 11, umfassend einen ersten Sensor und einen zweiten Sensor, der stromaufwärts des ersten Sensors angeordnet ist, und einen anderen zweiten Sensor, der stromabwärts des ersten Sensors angeordnet ist, sowie Mittel zum Vergleichen der Signale, die durch die Detektionsmittel der zwei zweiten Sensoren geliefert werden.

13. System zur Detektion nach einem der Ansprüche 10 bis 12, umfassend mehrere erste Sensoren, und bei dem die zweiten Sensoren in mehreren Gruppen verteilt sind, die in konzentrischer Weise um die ersten Sensoren herum angeordnet sind, wobei die Steuermittel der zweiten Sensoren derart ausgelegt sind, dass sie die Heizmittel derart steuern, dass die empfindlichen Zonen jeder Gruppe auf einer Temperatur sind, die von jenen der anderen Gruppen verschieden ist.

14. System zur Detektion nach einem der Ansprüche 8 bis 13, umfassend einen Kanal, in dem ein Sensor/Sensoren montiert ist/sind, und vorzugsweise umfassend eine mit dem Kanal verbundene Pumpe.

15. Verfahren zur Analyse einer Gasmischung, die wenigstens zwei Gassorten oder wenigstens zwei Familien von Gassorten umfasst, das einen Sensor nach einem der Ansprüche 1 bis 7 oder ein System zur Detektion nach einem der Ansprüche 8 bis 14 verwendet, umfassend wenigstens einen Zyklus, der die folgenden Schritte umfasst:

   a) Anlegen einer ersten Temperatur an wenigstens eine empfindliche Zone zum Bewirken einer Adsorption/Absorption von allen zu detektierenden Gassorten,
   b) Messen eines Signals zur Detektion der Adsorption/Absorption,
   c) dann Anlegen einer zweiten Temperatur zum Bewirken der Desorption eines Teils der Gassorten,
   d) Messen eines Signals zur Detektion der Desorption,
   e) Wiederholen der Schritte a), b), c) und d) mit Adsorptions-/ Absorptionstemperaturen, die verschieden sind von jener des vorhergehenden Schritts a), und bis alle interessierenden Sorten getrennt sind.

16. Analyseverfahren nach Anspruch 15, bei dem die Adsorptions-/ Absorptions- und Desorptionsbedingungen des folgenden Zyklus als Funktion der Signale bestimmt werden, die im Schritt d) erhalten werden, und/oder bei dem die Desorptionsbedingungen des Schritts c) als Funktion der Signale bestimmt werden, die im Schritt b) erhalten werden.

17. Analyseverfahren nach Anspruch 15 oder 16, umfassend wenigstens vor dem Schritt a) wenigstens einen Schritt der Reinigung durch Anlegen einer Temperatur, die eine Desorption derart bewirkt, dass sich die empfindliche Zone im Referenzzustand befindet.

18. Verfahren nach einem der Ansprüche 15 bis 17, bei dem das Analyseverfahren unterbrochen wird, wenn der Wert des Detektionssignals im Schritt b) kleiner als ein Schwellenwert ist.


**Claims**

1. A nanoelectromechanical and/or microelectromechanical gas sensor configured for the analysis of a mixture of at least two gaseous species or at least two families of gaseous species to be detected, said gas sensor comprising:

   - a fixed part (2),
   - at least one suspended part (4) in relation to fixed part (2),
   - at least one sensitive zone (6) carried on the suspended part (4), said sensitive zone (6) being able to adsorb/absorb and desorb gaseous species or families of gaseous species,
   - means for heating (8) of at least the sensitive zone (6),
   - means for detecting (10) the adsorption/absorption and the desorption of gaseous species or families of gaseous species, on the sensitive zone,
   - detection means for controlling (10) the heating means (8),

   **characterized in that** the detection means and the control means are configured to apply at least one cycle comprising the steps:

   a) application to at least one sensitive zone of a first temperature to provoke an adsorption/absorption of the gaseous species or families of every gaseous species to be detected,
   b) measurement of the adsorption/absorption detection signal,
   c) application of a second temperature to provoke the desorption of a part of the gaseous species or families of gaseous species,
   d) measurement of a detection signal of said desorption,
   e) repetition of steps a), b), c) and d) at adsorption/absorption temperature different from the adsorption/absorption temperature of a previous step a) and until all the gaseous species or families of gaseous species of interest are separated.

2. Gas sensor according to claim 1, comprising means for measuring the temperature of the sensitive zone (6).

3. Gas sensor according to claim 1 or 2, in which the suspended part (4) is mobile and the detection means comprise actuating means to place in movement the suspended part and means for measuring the movement of the suspended part, and in which the suspended part is at least in part an electrical conductor and the detection means measure a variation in the conductivity of the suspended part.

4. Gas sensor according to one of the claims 1 to 3, in which the heating means are Joule effect heating means, the suspended part being advantageously at least partially an electrical conductor and being intended to heat by Joule effect via circulation of a current.

5. Gas sensor according to claims 3 and 4, in which the heating means and the detection means are combined.

6. Gas sensor according to one of the claims 1 to 5, comprising a database of predefined temperature profiles.

7. Gas sensor according to one of the claims 1 to 6, having several suspended parts with sensitive zones of different surface areas and/or having properties of adsorption/absorption and desorption different from each other and/or the heating means are such that they are configured to heat different zones of the mobile part to different temperatures simultaneously.

8. A gas detection nanoelectromechanical and/or microelectromechanical system comprising at least a first gas sensor according to one of the claims 1 to 7, at least one second gas sensor and means for comparing and processing signals issuing from the detection means of each of said sensors, the sensors having advantageously different sensitive zones.

9. A gas detection system according to claim 8, in which the second gas sensor is a gas sensor according to one of the claims 1 to 7, the first gas sensor being placed in a reference environment and the second sensor being intended to be in contact with a gaseous mixture.

10. A gas detection system according to claim 8, in which the first gas sensor and the second gas sensor are intended to be placed in the same gaseous mixture, at least one sensitive zone of the first gas sensor being able to adsorb/absorb and desorb a first gaseous species or first family of gaseous species to be detected and the second gas sensor being a gas sensor according to one of the claims 1 to 7, at least one sensitive zone of the second sensor being able to adsorb/absorb and desorb a second gaseous species or second family of gaseous species to detect and in which

11. A gas detection system according to claim 8, comprising several second sensors arranged around a first sensor, the controller of the second sensors being such that they adsorb/absorb the gaseous species or families of gaseous species desorbed by the first sensor.

12. A gas detection system according to one of the claims 8 to 11, wherein a first sensor and a second sensor arranged upstream of the first sensor and another second sensor arranged downstream of the first sensor and with a means for comparing signals supplied by the detection means of the two second sensors.

13. A gas detection system according to one of the claims 10 to 12, having several first sensors and wherein the second sensors are spread in several groups arranged in a concentric manner around the first sensors, the controller of the second sensors being such that it controls the heater in such a way that the sensitive zone of each group is at a different temperature from those of the other groups.

14. A gas detection system according to one of the claims 8 to 13, comprising a channel wherein the sensor(s) is/are mounted, and advantageously comprising a pump connected to the channel.

15. An analysis method for analysis of a gaseous mixture of at least two gaseous species or at least two families of gaseous species implementing a sensor according to one of the claims 1 to 7 or a gas detection system according to one of the claims 8 to 14, comprising at least one cycle including the steps:

a) application to at least one sensitive zone of a first temperature to provoke an adsorption/absorption of the gaseous species or families of every gaseous species to be detected,

b) measurement of the adsorption/absorption detection signal,

c) application of a second temperature to provoke the desorption of a part of the gaseous species or families of gaseous species,

d) measurement of a detection signal of said desorption,

e) repetition of steps a), b), c) and d) at adsorption/absorption temperature different from the adsorption/absorption temperature of a previous step a) and until all the gaseous species or families of gaseous species of interest are separated.

16. An analysis method according to claim 15, wherein the adsorption/absorption and desorption conditions of the following cycle are determined in relation to the signals obtained in step d) and/ot the desorption conditions of step c) are determined in relation to the signals obtained in step b).

17. An analysis method according to claim 15 or 16, wherein at least prior to step a), at least one cleaning step is carried out by application of a temperature provoking a desorption such that the sensitive zone returns to its reference state.

18. A analysis method according to one of the claims 15 to 17, in which when the value of the detection signal of step b) is less than a threshold value, the analysis procedure is interrupted.

FIG.1

FIG.2A

FIG.2B

FIG.2C

FIG.2D

FIG.2E

FIG.2F

FIG.3

FIG.4

C1

C2

FIG.5

G4

G3

G1

G2

FIG.6

FIG.7

FIG.8

FIG.9

FIG.10

FIG.11A

FIG.11B

FIG.11C

FIG.11D

FIG.11E

FIG.11F

FIG.11G

FIG.11H

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0140793 A

- US 20050090018 A

**Littérature non-brevet citée dans la description**

- **MATTHEW KASPER.** *SIMULTANEOUS NANO-THERMAL ANALYSIS USING HEATED MICRO-CANTILEVERS,* 30 Avril 2010

- Knudsen pump driven by a thermoelectric material. **KUNAL PHARAS ; SHAMUS MCNAMARA.** Journal of Micromechanics and Microengineering. 2010 IOP Publishing Ltd, 29 Novembre 2010, vol. 20